Europäisches Patentamt

European Patent Office

Office européen des brevets

(19)

(11) Veröffentlichungsnummer: **0 386 408**
**A1**

(12) **EUROPÄISCHE PATENTANMELDUNG**

(21) Anmeldenummer: **90100681.7**

(22) Anmeldetag: **13.01.90**

(51) Int. Cl.5: **A61M 25/00**

(30) Priorität: **09.03.89 DE 3907618**

(43) Veröffentlichungstag der Anmeldung:
**12.09.90 Patentblatt 90/37**

(84) Benannte Vertragsstaaten:
**AT BE CH DE DK ES FR GB GR IT LI LU NL SE**

(71) Anmelder: **B. Braun Melsungen AG**
**Carl-Braun Strasse**
**D-3508 Melsungen(DE)**

(72) Erfinder: **Haindl, Hans-Günter, Dr.**
**Schöne Aussicht 4**
**D-3508 Melsungen(DE)**

(74) Vertreter: **Selting, Günther, Dipl.-Ing. et al**
**Patentanwälte von Kreisler, Selting, Werner**
**Deichmannhaus am Hauptbahnhof**
**D-5000 Köln 1(DE)**

(54) **Katheter.**

(57) Bei einem Katheter (10) sind in einem Katheterschlauch (11) zwei nebeneinanderliegende Lumina (13,14) durch ein längsverlaufendes Septum (12) voneinander getrennt. Dabei weist das erste Lumen (13) an der Spitze des Katheterschlauches (11) eine endständige Öffnung (20) (Ausstoßöffnung) auf und axial aufeinanderfolgende seitliche Öffnungen (22,23) (Ansaugöffnungen) des zweiten Lumens (14) sind durch Permanentdeformation des Katheterschlauches (11) in verschiedene Ebenen verlegt. Auf diese Weise wird die Gefahr der gleichzeitigen Okklusion aller Ansaugöffnungen durch Anlegen an die Gefäßwand behoben.

Vorzugsweise ist der Katheterschlauch (11) im Längsbereich der seitlichen Öffnungen (22,23) des zweiten Lumens (14) um seine Längsachse um mindestens 90° verdreht.

FIG.2

EP 0 386 408 A1

## Katheter

Die Erfindung betrifft einen Katheter mit zwei in einem Katheterschlauch nebeneinanderliegenden Lumina, die durch ein längsverlaufendes Septum voneinander getrennt sind, bei dem dem ersten Lumen eine endständige Öffnung an der Spitze des Katheterschlauches und dem zweiten Lumen axial aufeinanderfolgende seitliche Öffnungen in der Wand des Katheterschlauches zugeordnet sind.

Derartige doppellumige Katheter werden für die Hämodialyse/Hämofiltration benutzt, die der in-vivo-Reinigung von Blut dient, wobei aus einem Blutgefäß abgepumptes Blut durch das eine Lumen abgezogen und z.B. intensiver Ultrafiltration unterzogen wird, und das Retentat gegebenenfalls zusammen mit Substitutflüssigkeit durch das andere Lumen wieder in das Blutgefäß zurückgeführt wird. Solche doppellumigen Katheter haben den Vorteil, daß man die Hämodialyse/Hämofiltration mit nur einer Punktion einer zentralen Vene durchführen kann. Bei einem derartigen bekannten Katheter (EP-A-168 136) hat das erste der beiden Lumina, die durch ein zentrales Septum im Katheterschlauch gebildet sind, eine endständige Öffnung und einige seitliche Öffnungen, die auf einer geraden Linie liegen. Die seitlichen Öffnungen des zweiten Lumens sind einige Zentimeter von der Spitze entfernt ebenfalls auf einer geraden Linie gegenüber den seitlichen Öffnungen des ersten Lumens angeordnet. Über die von der Spitze weiter entfernten seitlichen Öffnungen des zweiten Lumens wird das Blut angesaugt und über die der Spitze näherliegenden Öffnungen des ersten Lumens wird das gereinigte Blut zurückgegeben. Der Katheter kann nach der Seldinger-Technik eingeführt werden, d.h. er kann mit einem Drahtmandrin, der zunächst durch einen Hauttunnel zum Zielort vorgeschoben wird und der als Leitschiene für den aufgeschobenen Doppellumen-Katheter dient, verlegt werden. Auch läßt er sich durch eine Punktionshohlnadel einführen. Durch seine Flexibilität paßt sich der Katheterschlauch bei der Verlegung leichten Krümmungen der Vene an. Gewünschtenfalls kann er von Hand in eine erwartete einfache Krümmung vorgebogen werden. Da bei diesem doppellumigen Katheter die seitlichen Öffnungen des zweiten Lumens alle auf gerader achsparalleler Linie aufeinanderfolgend an einer Seite des Katheters liegen, besteht die Gefahr, daß trotz des Vorhandenseins mehrerer Öffnungen der Katheterschlauch sich mit dieser Seite an die Gefäßwand anlegt und es zur Okklusion der Ansaugöffnungen kommt. Dieser Gefahr kann nicht durch die erwähnte manuelle Verbiegung des Katheterschlauches begegnet werden, weil die Biegung nicht dauerhaft ist und der die Öffnungen gegen die Gefäßwand anziehenden Ansaugkraft nicht entgegenwirken kann.

Bei einem anderen bekannten doppellumigen Katheter (EP-A-101 890) ist zur Verhinderung der Okklusion der Ansaugöffnungen zwischen zwei auf gerader achsparalleler Linie aufeinanderfolgenden Ansaugöffnungen des zweiten Lumens auf der Außenfläche der Katheterwand ein Außenwulst angeordnet, der sich etwa über den halben Außenumfang des Katheterschlauches erstreckt und in bezug auf die Gefäßwand als Abstandshalter dienen soll. Das zweite Lumen ist kürzer als das erste Lumen und es endet durch Wandanschluß des Septums in beträchtlichem Abstand von der Spitze des Katheterschlauches, die geschlossenen ist. Das erste Lumen erstreckt sich somit bis zur geschlossenen Spitze des Katheterschlauches und hat im Spitzenbereich den vollen Durchmesser des Katheterschlauches. Seine Öffnungen (Ausstoßlöcher) sind in diesem Bereich über den Umfang des Katheterschlauches verteilt. Die Herstellung eines solchen Katheters ist kostspielig. Infolge des Fehlens einer endständigen Öffnung ist dieser Katheter nicht mit einem Führungsdraht verlegbar. Auch eine Einführung durch eine Punktionshohlnadel ist nicht möglich, weil der Außenwulst den Vorschub in der Nadel behindert; der Katheter muß operativ eingebracht werden, was wegen starker Belastung des Patienten unerwünscht ist.

Zur Verhinderung des Verschlusses seitlicher Ansaugöffnungen des einen Lumens eines mehrlumigen Katheters sind Koaxial-Katheter entwickelt worden (DE-A-28 13 275), bei denen die Lumina nicht nebeneinander, sondern koaxial zueinander angeordnet sind. Ein innerer Katheter mit endständiger Öffnung wird von einem äußeren Katheter unter Bildung eines Ringlumens umgeben, das durch umfangsmäßig und axial verteilte Öffnungen in dem äußeren Katheter zugänglich ist. Der äußere Katheter endet im Abstand von der spitzenseitigen Mündung des inneren Katheters und umfaßt diesen mit einem verjüngten Endabschnitt abdichtend. Die umfangsmäßige Verteilung der seitlichen Öffnungen sorgt zwar dafür, daß immer die Ansaugöffnungen auf der der Gefäßwand abgewandten Seite offen sind, jedoch sind Koaxial-Katheter für die Hämodialyse/Hämofiltration weniger günstig, weil sie wegen der Addition der Wandstärken des inneren und des äußeren Katheters ein schlechteres Verhältnis zwischen Durchfluß und Außendurchmesser haben als Katheter, deren nebeneinanderliegende Lumina sich durch Teilung des Lumens des Katheterschlauches mit Hilfe eines Septums ergeben.

Der Erfindung liegt die Aufgabe zugrunde, einen Doppellumen-Katheter der eingangs erwähnten Art so auszubilden, daß die Gefahr der gleichzeitigen Okklusion aller Ansaugöffnungen des zweiten Lumens in der Wand des Katheterschlauches durch Anlegen an die Gefäßwand behoben ist.

Diese Aufgabe wird erfindungsgemäß dadurch gelöst, daß die seitlichen Öffnungen des zweiten Lumens durch Permanentdeformation des Katheterschlauches auf eine ungerade Linie verlegt sind.

Auf diese Weise gelingt es, bei einem außen glattwandigen doppellumigen Seldinger-Katheter, dessen Lumina nebeneinanderliegen und - im Querschnitt gesehen - je eine Hälfte des Katheterschlauches einnehmen, immer einige der seitlichen Öffnungen des zweiten Lumens von der Gefäßwand entfernt zu halten, so daß sie während des Absaugens von Blut nicht an die Gefäßwand angesogen werden und sich nicht verschließen können. Der Katheterschlauch ist außen vorsprungslos und die endständige Spitze seines ersten Lumens ermöglicht eine Verlegung mit Hilfe eines Führungsdrahtes nach der Seldinger-Technik. Die Art der Einbringung des Katheters und seine optimale Durchflußcharakteristik, sowohl im ersten Lumen beim Wiedereinführen des Retentats als auch beim zweiten Lumen bei Ansaugen des zu behandelnden Blutes, machen diesen Katheter für die Hämodialyse/ Hämofiltration besonders geeignet, weil er patientenschonend und zuverlässig ist.

In vorteilhafter Ausgestaltung der Erfindung ist der Katheterschlauch im Längsbereich der seitlichen Öffnungen des zweiten Lumens um seine Längsachse um mindestens 90° verdreht. Zur Torsion des Katheterschlauches wird vorzugsweise der Katheterschlauch torquiert eingespannt und zur Erreichung eines Memory-Effektes wärmebehandelt. Die Torsionsverformung bleibt stabil und die seitlichen Löcher des zweiten Lumens sind über den Umfang des Katheterschlauches gestaffelt verteilt. Auf diese Weise wird der prinzipielle Nachteil eines zweilumigen Katheters mit nebeneinanderliegenden Lumina, nämlich daß alle Sauglöcher auf dem Axialbereich einer Umfangshälfte des Katheterschlauches und damit nur auf einer Seite liegen, behoben. Vorzugsweise ist der Erstreckungswinkel der Reihe von Öffnungen größer als 180°.

Bei einer anderen vorteilhaften Ausgestaltung der Erfindung ist der Katheterschlauch im Längsbereich der seitlichen Öffnungen des zweiten Lumens in der Ebene des Septums wellenförmig gebogen. In diesem Falle liegen die Öffnungen zwar linear aufeinanderfolgend auf einer Seite des Katheterschlauches, aber sie sind auf Wellenberge und Wellentäler des gewellten Katheterschlauches verteilt, so daß immer einige Öffnungen von der Gefäßwand entfernt gehalten sind. Eine Kombination eines torquierten und wellenförmig gebogenen Katheterschlauches ist möglich.

Weiterhin kann der Katheterschlauch im Längsbereich der seitlichen Öffnungen des zweiten Lumens räumlich schraubenförmig gewendelt sein. Auch in diesem Falle ist gewährleistet, daß einige seitliche Öffnungen des zweiten Lumens aus der Ebene der Gefäßwand verlegt sind und sich keine Okklusion der Ansaugöffnungen ergeben kann.

Das zweite Lumen ist an seinem proximalen (patientennahen) Ende neben der endständigen Öffnung des ersten Lumens verschlossen und es ist der Totraum des zweiten Lumens bis zu seiner ersten spitzennahen seitlichen Öffnung mit elastischem Füllmaterial ausgefüllt, damit sich in dem Totraum kein Blut ansammelt. Das Septum verläuft also von einem zum anderen Ende des Katheterschlauches, wodurch seine Herstellung und der Verschluß des zweiten Lumens erleichtert werden. Zusätzlich zu der endständigen Öffnung des ersten Lumens kann in seiner Umfangszone in der Wand des Katheterschlauches mindestens eine weitere Öffnung im Bereich der Spitze angeordnet sein, um die Ausströmleistung des Katheters zu erhöhen. Vorzugsweise beträgt der Abstand zwischen einer solchen seitlichen Öffnung des ersten Lumens und der ersten Öffnung des zweiten Lumens etwa 2 bis 3 cm zwischen den gegeneinander gerichteten Rändern der beiden Öffnungen, die sich auf entgegengesetzten Seiten des Katheterschlauches befinden.

In den Zeichnungen sind Ausführungsbeispiele der Erfindung schematisch dargestellt.

Es zeigen:

Fig. 1 eine Draufsicht auf einen torquierten Katheter,

Fig. 2 das patientenseitige Ende des Katheters nach Fig. 1 in vergrößertem Maßstab,

Fign. 3 bis 5, Querschnitte längs der Linien III-III, IV-IV und V-V in Fig. 2,

Fig. 6 eine Draufsicht auf einen in einer Ebene gewellten Katheter, und

Fig. 7 eine Draufsicht auf einen räumlich schraubenförmig gewendelten Katheter.

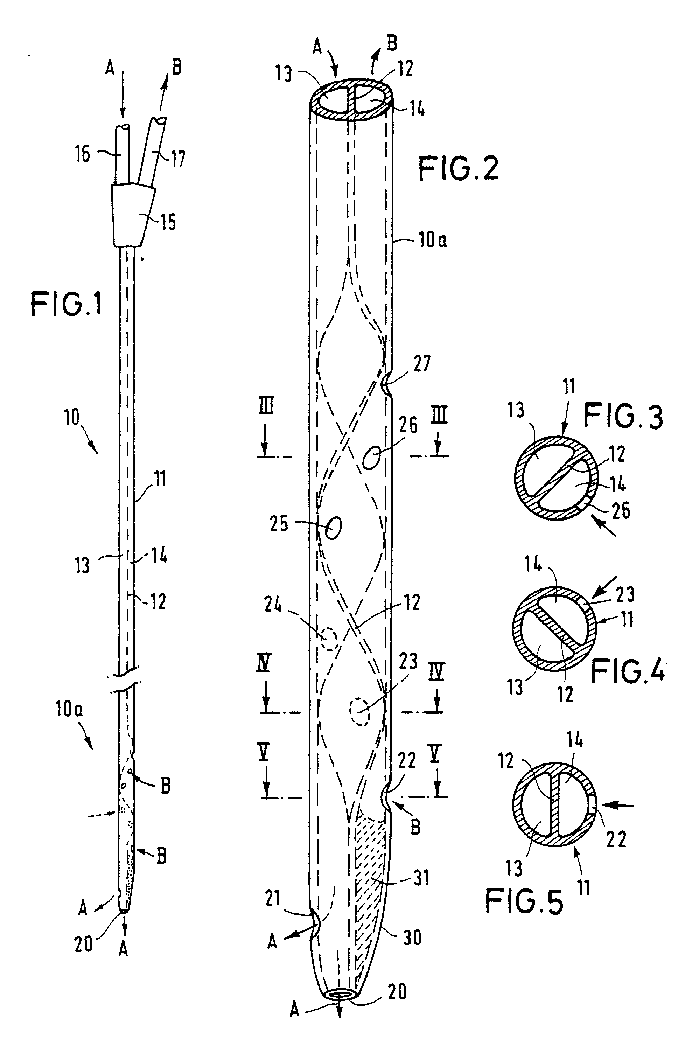

Der Katheter 10 gemäß Fign. 1 bis 5 besteht im wesentlichen aus einem Katheterschlauch 11 aus weichem biegbaren, jedoch knickanfälligem Polyurethanmaterial. Der kreiszylindrische Innenraum des Katheterschlauches 11 ist durch ein quergerichtetes Septum 12, das sich von einem Ende des Katheterschlauches 11 bis zum anderen erstreckt, in zwei halbzylindrische Lumina 13 und 14 unterteilt, die miteinander keine Verbindung haben und von denen das Lumen 13 als erstes Lumen und das Lumen 14 als zweites Lumen bezeichnet wird. An dem patientenfernen Ende ist ein Katheteransatz 15 befestigt, der zwei Kanäle enthält, die eine Verbindung zwischen den beiden Lumina 13 und 14 mit vorzugsweise flexiblen Schlauchleitungen 16 und 17 herstellen. Die Schlauchleitung 17 dient als an eine Pumpe angeschlossene Absaugleitung, während die Schlauchleitung 16 als Rückführleitung für filtriertes Blut mit Substitutflüssigkeit dient. Die Pfeile A und B geben den jeweiligen Flüssigkeitsverlauf an.

Der patientenseitige Spitzenteil 10a des Katheters 10 weist Öffnungen auf, die den Lumina 13 und 14 zugeordnet sind. Das Lumen 13 ist mit einer endständigen Öffnung 20 an der Spitze 30 des Katheterschlauches 11 ausgestattet. Zusätzlich ist ihm eine seitliche Öffnung 21 zugeordnet, die in der Nähe der endständigen Öffnung 20 vorgesehen ist und dem Auslaß von Retentat dient. Das Lumen 14 ist durch eine Reihe von seitlichen Öffnungen 22 bis 27 für den Blutstrom B zugänglich. Die der Spitze des Katheterschlauches 11 nächstgelegene Öffnung 22 des zweiten Lumens 14 hat zu der gegenüberliegenden Öffnung 21 des ersten Lumens 13 einen Abstand von etwa 2 bis 3 cm - gemessen von den einander zugewandten Rändern der Öffnungen - damit die Stellen der Ansaugung und des Ausstoßens des Blutes genügend weit voneinander entfernt sind, um eine Rezirkulation des gereinigten Blutes auszuschließen. Das zweite Lumen 14 ist am spitzenseitigen Ende 30 des Katheterschlauches 11 geschlossen. Der Totraum zwischen der seitlichen Öffnung 22 und dem Endverschluß ist mit flexiblem Material 31 ausgefüllt, damit sich in diesem Totraum des Lumens 14 keine Blutrückstände ansammeln.

Die Öffnungen 22 bis 27 in der dem Lumen 14 zugehörigen Wand des Katheterschlauches 11 liegen auf einer Schraubenlinie, die einen Umfangsbereich von ca. 360° erfaßt. Diese Schraubenlinien-Konfiguration ergibt sich dadurch, daß der Katheterschlauch 11 im Bereich des Spitzenteiles 10a um seine Längsachse verdreht ist und durch Wärmebehandlung in der gewollten Verdrehposition bleibt. Bei Verlegung des Katheters 10 in einem Blutgefäß, z.B. einer Vene, liegen auf diese Weise immer nur wenige Öffnungen gegen die Gefäßwand an und die anderen Öffnungen sind frei und offen, so daß eine Okklusion der Ansaugöffnungen nicht zu befürchten ist. Wenn beispielsweise die Gefäßwand sich an die Öffnungen 22 und 27 anlegt, bleiben die Öffnungen 23 bis 26 von der Gefäßwand frei und sorgen für gleichmäßige Menge des abgesaugten Blutes. Die Torquierung des Septums 12 im Spitzenteil 10a des Katheters 10 ist für den Blutstrom A und für den Blutstrom B nicht restriktiv. Aufgrund der Weichheit des Kathetermaterials ist sie auch für einen Führungsdraht kein Hindernis, so daß der Katheter 10 mit einem als Leitschiene dienenden Führungsdraht nach der Seldinger-Technik in eine Punktionsöffnung eines Gefäßes vorsichtig und gezielt vorgeschoben werden kann.

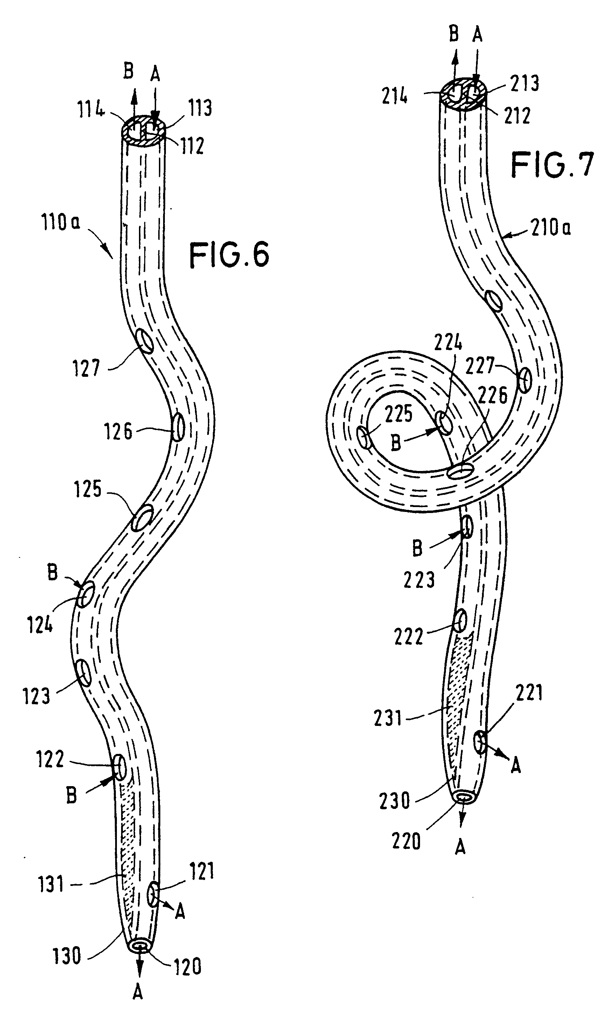

Von den Kathetern der Beispiele nach Fign. 6 und 7 sind nur die patientennahen gelochten Spitzenteile 110a und 210a dargestellt. Der Spitzenteil 110a des Beispiels nach Fig. 6 ist in der Ebene des Septums 112 gewellt. Die linear aufeinanderfolgenden seitlichen Öffnungen 122 bis 127 des zweiten Lumens 114 sind auf diese Weise so verlegt, daß zumindest die in den Wellentälern befindlichen Öffnungen 122,123 und 125,126 nicht gegen eine Gefäßwand anliegen und zum Ansaugen von Blut B offenbleiben. Der Totraum zwischen dem verschlossenen Ende 130 des Lumens 114 und der ersten Öffnung 122 ist mit flexiblem Material 131 ausgefüllt, das die Spitze versteift und die Einführung des Katheters erleichtert. Auch wird der Gefahr des Kollabierens des Katheters im Bereich seiner Spitze beim Ansaugen von Blut durch die Füllung 131 entgegengewirkt.

Die Wand des anderen Lumens 113, das der Zuführung gereinigten Retentats A dient, ist im wesentlichen über die gesamte Länge geschlossen. Lediglich eine endständige Öffnung 120 und eine zusätzliche seitliche Öffnung 121 sind vorhanden. Der Abstand der Öffnung 121 zu der ersten Öffnung 122 des anderen Lumens 114 beträgt ebenfalls etwa 2 bis 3 cm.

Bei dem Katheter nach Fig. 7, dessen Kanal durch das Septum 212 in ein erstes Lumen 213 und ein zweites Lumen 214 unterteilt wird, ist der Spitzenteil 210a schraubenförmig gewendelt, so daß die dem zweiten Lumen 214 zugeordneten seitlichen Öffnungen 222 bis 227 nicht in einer Ebene liegen, sondern so zueinander versetzt sind, daß immer einige dieser Öffnungen 222 bis 227 von der Gefäßwand frei und zum Durchlaß von angesaugtem Blut B offen sind. Der Abstand zwischen den Öffnungen 222 und einer seitlichen Öffnung 221 des ersten Lumens 213 beträgt auch in diesem Falle etwa 2 bis 3 cm. Eine endständige Öffnung 220 dient dem Auslaß von gereinigtem Blut A. Das Lumenstück zwischen dem verschlossenen Ende 230 und der ersten Öffnung 222 ist mit flexiblem Material 231 verfüllt.

Die Wellung bzw. Wendelung der Spitzenteile 110a bzw. 210a der beiden erläuterten Ausführungsbeispiele nach Fign. 6 und 7 behindern das Auffädeln der Katheter auf einen Führungsdraht nicht, so daß auch diese profilierten Katheter mit Hilfe der Seldinger-Technik problemlos verlegbar sind.

## Ansprüche

1. Katheter mit zwei in einem Katheterschlauch (11) nebeneinanderliegenden Lumina (13,14), die durch ein längsverlaufendes Septum (12) voneinander getrennt sind, bei dem dem ersten Lumen (13) eine endständige Öffnung (20) an der Spitze des Katheterschlauches (11) und dem zweiten Lumen (14) axial aufeinanderfolgende seitliche Öffnungen (22,23) in der Wand des Katheterschlauches (11) zugeordnet sind,

**dadurch gekennzeichnet,**

daß die seitlichen Öffnungen (22,23) des zweiten Lumens (14) durch Permanentdeformation des Katheterschlauches (11) auf eine ungerade Linie verlegt sind.

2. Katheter nach Anspruch 1, dadurch gekennzeichnet, daß der Katheterschlauch (11) im Längsbereich der seitlichen Öffnungen (22,23) des zweiten Lumens (14) um seine Längsachse um mindestens 90° verdreht ist.

3. Katheter nach Anspruch 1 oder 2, dadurch gekennzeichnet, daß der Katheterschlauch im Längsbereich der seitlichen Öffnungen (122,123) des zweiten Lumens (114) in der Ebene des Septums (112) wellenförmig gebogen ist.

4. Katheter nach Anspruch 1 oder 2, dadurch gekennzeichnet, daß der Katheterschlauch im Längsbereich der seitlichen Öffnungen (222,223) des zweiten Lumens (214) räumlich schraubenförmig gewendelt ist.

5. Katheter nach einem der Ansprüche 1 bis 4, dadurch gekennzeichnet, daß das zweite Lumen (14) an seinem proximalen Ende (30) neben der endständigen Öffnung (20) des ersten Lumens (13) verschlossen ist und daß der Totraum des zweiten Lumens (14) bis zu seiner ersten spitzennahen seitlichen Öffnung (22) mit elastischem Füllmaterial (31) ausgefüllt ist.

FIG.1

FIG.2

FIG.3

FIG.4

FIG.5

FIG.6

FIG.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y,D | EP-A-0 168 136 (SHILEY INC.)<br>* Anspruch 1; Abbildungen 1,2 *<br>--- | 1-5 | A 61 M 25/00 |
| Y | US-A-4 795 439 (R.L. GUEST)<br>* Zusammenfassung; Abbildung 2 *<br>--- | 1,2,5 | |
| Y | EP-A-0 132 344 (PURDUE RESEARCH FOUNDATION)<br>* Seite 3, Zeilen 25-30; Ansprüche 1,2; Abbildungen 1-5 *<br>--- | 3,4 | |
| A | US-A-2 492 384 (A.L. KASLOW)<br>* Seite 3, Zeilen 19-32; Abb. *<br>--- | 1-3 | |
| A | US-A-4 643 716 (G.W. DRACH)<br>* Ansprüche 1-3; Abbildung 1 *<br>--- | 3-4 | |
| A | DE-U-8 805 515 (VYGON GmbH)<br>* Anspruch 5; Abbildung 3 *<br>----- | 5 | |

**RECHERCHIERTE SACHGEBIETE (Int. Cl.5)**

A 61 M

Der vorliegende Recherchenbericht wurde für alle Patentansprüche erstellt

| Recherchenort | Abschlußdatum der Recherche | Prüfer |
|---|---|---|
| DEN HAAG | 06-06-1990 | VILLENEUVE J-M.R.J. |